# EUROPEAN PATENT APPLICATION

(11) **EP 1 657 257 A1**
(43) Date of publication of application: **17.05.2006**
(21) Application number: 04026819.5
(22) Date of filing: 11.11.2004
(51) Int. Cl.: C07K 19/00, C07K 14/705

(54) **Recombinant TLR-MD-2 fusion proteins**

(71) Applicant: Klinikum der Universität Regensburg, 93042 Regensburg (DE)
(72) Inventor: Brandl, Katharina, 93047 Regensburg (DE); Falk, Werner, Prof. Dr. med., 93152 Schönhofen (DE)
(74) Representative: Graf von Stosch, Andreas

(57) **Abstract**

The present invention provides recombinant TLR-MD-2 fusion proteins capable of binding to lipopolysaccharide (LPS) containing a TLR-moiety comprising the extracellular domain of a Toll-like receptor (TLR) or a functional derivative thereof, fused with a MD-2-moiety comprising MD-2 or a functional derivative thereof. In a preferred embodiment of the invention, the TLR-moiety and MD-2-moiety are fused via a flexible or inflexible linker and are provided with IgG-Fc and/or His-domains. The TLR-MD-2 fusion proteins of the present invention enable regulating of lipopolysaccharide (LPS) activity and modulating signaling of the Toll-like receptor (TLR) allowing an intervention in the septic process at its earliest state.

## Description

### Field of the Invention

The present invention relates to the field of modulation and regulation of immune responses of vertebrates. More particularly, this invention provides recombinant fusion proteins for blocking lipopolysaccharide (LPS) activity and modulating signaling of the Toll-like receptor (TLR).

### Background of the invention

The evolutionary conserved Toll-like receptor (TLR) family as specific pathogen recognition molecules and signal transducers is critical for the activity of the innate immune system. TLR-induced signaling is involved in infectious diseases, chronic inflammatory diseases and sepsis. Sepsis is a life threatening disorder with approximately 450.000 cases occurring in the United States every year. About one third of affected patients will die.

Once microbes enter the organism, innate immunity is immediately activated as the first line of defense. The innate immune system coordinates the defensive operations by recognition of the invading microbes and release of cytokines that subsequently stimulate macrophages and natural killer cells. The latter may effectively kill pathogens, but they also activate the adaptive immune system by the secretion of cytokines and co-stimulatory molecules in order to enhance B- and T-lymphocyte responses. It is assumed that in sepsis this concerted action of the innate and acquired arms of the immune system gets out of control eventually resulting in multiple organ failure and death.

The discovery of the Toll-like receptors (TLRs) has been a milestone in the understanding of the molecular mechanisms in the pathogenesis of sepsis. TLRs specifically recognize different microbial molecules and are important for the activation of the innate immune system. Up to date 10 distinct TLRs have been identified in humans whereas in mice a new member, TLR11, was recently described, which specifically recognizes a component of uropathogenic bacteria.

All TLRs are type I integral membrane receptors with extracellular leucine rich regions and highly homologous Toll/IL-1 R (TIR) cytoplasmic domains. The differences in their leucine-rich repeats of the extracellular domains are critical for recognizing a large variety of different microbial structures. Signaling occurs via the TIR domains and a family of adapter molecules which couple to NF- 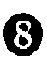 B and IRF3 pathways.

Among all TLRs, TLR4, which recognizes Gram-negative cell wall structures, has been the most intensively studied. LPS is the principal component of the outer membrane of Gram-negative bacteria and it adapts via serum and cell-surface proteins, including LPS binding protein and CD14, to the TLR4/MD-2 signaling complex. TLR4 is the signaling subunit of the LPS receptor, which is strictly dependent upon the presence of MD-2, a small cysteine-rich glycoprotein, that binds to the ectodomain of TLR4 and to LPS.

TLR2 is the Gram-positive immunostimulatory counterpart and mediates the response to a diverse set of molecular structures, including peptidoglycan, lipoteichoic acid (LTA), lipoarabinomanan, bacterial lipoproteins, as well as some variants from Gram-negative bacteria, yeast, spirochetes and fungi. The recognition of this broad spectrum of molecules by TLR2 is achieved at least in part by heteromerization with either TLR1 or TLR6, in contrast to TLR4, which seems to function as a homodimer. As it has been shown recently, stimulation of TLR4 leads to its aggregation into large complexes.

Although much is known about the molecular mechanisms leading to sepsis, septic shock and systemic inflammatory response, successful treatments to decrease sepsis related morbidity and mortality have remained an elusive goal.

Aiming for this goal, several clinical trials have been conducted to control signaling via TLR4. However, approaches like anti-endotoxin antibodies, anti-endotoxin vaccine strategies, agents that inhibit endotoxin synthesis, blockade of LPS-binding protein and recently the use of anti-CD14 antibodies have not been successful so far (Opal, S. M. and Gluck, T. 2003. Endotoxin as a drug target. Crit Care Med. 31, p57-64; Reinhart, K. et al. 2004. CD14 receptor occupancy in severe sepsis: results of a phase I clinical trial with a chimeric CD14 monoclonal antibody (IC14). Crit Care Med. 32, p1100-1108.)

Thus, careful control of TLR signaling at the receptor level is required to balance the beneficial and harmful effects of bacterial products. It is necessary not to abolish the important recognition of microbial products by TLRs, but to prevent the overwhelming challenge by these same products, which is assumed to be responsible for the overreaction of innate immunity once signal transduction has occurred in the case of sepsis.

Therefore, it is the object of the present invention to provide a means of regulating lipopolysaccharide (LPS) activity and modulating signaling of the Toll-like receptor (TLR) allowing an intervention in the septic process at its earliest state.

### Summary of the Invention

Consequently, this invention provides in a first aspect a recombinant TLR-MD-2 fusion protein capable of binding to lipopolysaccharide (LPS) containing a TLR-moiety comprising the extracellular domain of a Toll-like receptor (TLR) or a functional derivative thereof, fused with a MD-2-moiety comprising MD-2 or a functional derivative thereof. In a preferred embodiment of the invention, the TLR-moiety and MD-2-moiety are fused via a flexible or inflexible linker and are provided with a IgG-Fc and/or His-domain.

Furthermore, the present invention provides a nucleic acid molecule encoding the recombinant TLR-MD-2 fusion protein and a method for preparing the recombinant TLR-MD-2 fusion protein comprising inserting a nucleic acid coding for the recombinant TLR-MD-2 fusion protein into an expression vector, transfecting said expression vector into host cells and recovering the produced TLR-MD-2 fusion protein from the cell culture.

In another aspect the present invention provides a pharmaceutical composition containing a recombinant TLR-MD-2 fusion protein or fusion protein homopolymer or heteropolymer complex as active ingredient which is used for the prophylactic and/or therapeutic treatment of allergic reactions, infections diseases and inflammatory conditions, such as sepsis.

### Brief Description of the Figures

Figure 1 is a schematic representation of an embodiment of the inventive fusion protein consisting of a TLR-domain and a MD-2-domain linked by a flexible linker and provided with IgG-Fc-tail to form a fusion protein dimer *(LPS-Trap-Fc).*
Figure 2 shows an Anti-Flag Western-blot analysis verifying the expression of T4Fc in stably transfected S2-cells.
Figure 3 shows the effect of T4Fc on TLR4 activation.
Figure 4 shows the blocking of LPS-mediated IL-6 production by the T4Fc/MD-2 complex in RAW 264.7 cells.
Figure 5 shows an Anti-Flag Western-blot analysis demonstrating the expression of *LPS-Trap-Fc* and *LPS-Trap* in S2-cells.
Figure 6 shows the results of an Anti-Flag Western-blot analysis verifying the binding of LPS to *LPS-Trap.*

### Detailed Description of the Invention

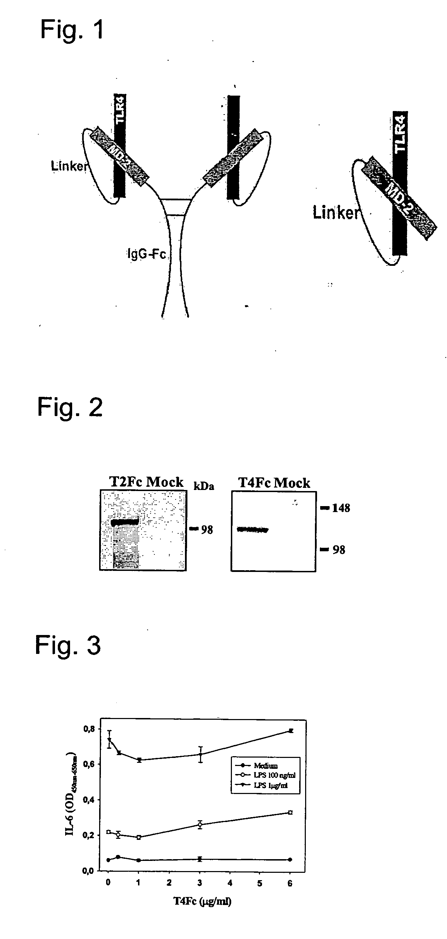

Figure 1 shows a preferred embodiment of the inventive TLR-MD-2 fusion protein consisting of a TLR-moiety and a MD-2-moiety linked by a flexible linker and provided with a IgG-Fc-tail to form a fusion protein dimer *(LPS-Trap-Fc).*

The TLR-moiety comprises the extracellular domain of a Toll-like receptor (TLR) or a functional derivative thereof and the MD-2-moiety comprises the MD-2 protein or a functional derivative thereof.

In one embodiment, the domains of the TLR-MD-2 fusion protein of the invention are connected directly to each other. In preferred embodiments, as shown in Fig. 1, a linker sequence may be included between the components, which may comprise one or more molecules, such as amino acids. This linker sequence serves for the structural delimitation of the various functional components in the recombinant fusion protein and can preferably also exert a "hinge" function, i.e. an amino acid sequence of flexible structure. Any known flexible or inflexible amino acid sequence which is suitable to link different proteins can be used. For example, a linker sequence may include one or more amino acids naturally connected to the domain component. A linker sequence may also include a sequence used to enhance expression of the fusion protein, provide restriction sites, allow component domains to form optimal tertiary structures and/or to enhance the interaction of a component with its target molecule. The linker may also comprise at least one proteolytic cleavage site, which enables the TLR-domain to be separated from the MD-2-domain. In one embodiment, the fusion protein of the invention comprises a linker of 1-25 amino acids. Preferably, the linker comprises 3 to 15, more preferably, 6 to 10, amino acids, and is preferably composed of at least 50 % up to 70 % of glycine residues, whereby the remaining residues are preferably composed of proline.

In a preferred embodiment, as shown in Fig. 1, the C-terminus of the fusion protein construct is provided with a IgG-Fc-domain which can mediate dimerization or polymerization of several TLR-MD-2 fusion proteins via disulfide bonds. The IgG-Fc immunoglobulin domain may be selected from IgG1, IgG2, IgG3 or IgG4 or a modified immunoglobulin domain derived therefrom. To control a potential *in vivo* activity of the IgGFc-moiety using of an inactive IgGFc element may be preferable.

Additionally, the fusion protein may comprise additional sequence segments. Sequences which are preferred for the purposes of the present invention are, in this context, those known as tag sequences, for example at least one flag tag, that is to say the amino acid sequence DYKDDDDK, and/or else for example at least one His tag (comprising several consecutive histidines, for example at least five, preferable His6) and/or further tag sequences or antigenic sequences. The tag sequences may occur at any position of a recombinant fusion protein according to the invention, preferably at the C- terminus.

Further embodiments may include a signal sequence at the amino-terminus of the TLR-MD-2 fusion protein of the invention which allows secretion from host cell after recombinant expression. Such a signal sequence may be native to the cell, recombinant, or synthetic.

The components of the TLR-MD-2 fusion protein of the invention may be arranged in a variety of configurations. For example, the MD-2 domain may be arranged at the amino-terminus of the fusion protein and may be followed by the TLR-domain at the carboxyl-terminus of the fusion protein. In a preferred embodiment as shown in Fig.1, the TLR-domain is located at the amino-terminus of the fusion protein whereas the MD-2-domain is arranged at the carboxyl-terminus.

The TLR-moiety of the fusion protein of the present invention can consist in any known extracellular domain of TLR, i. e., TLR1 to TLR11 or a functional derivative thereof. Preferred TLR-MD-2-fusion proteins contain the extracellular domain of TLR4 or TLR2 or a functional derivative thereof.

The term functional derivative refers in particular to those proteins which maintain the biological function, in particular the binding property with the interaction partner, but whose sequence shows differences to the corresponding native sequences. These sequence deviations may take the form of one or more insertion(s), deletion(s) and/or substitution(s), a sequence homology of at least 70% being preferred and a sequence homology of at least 85% between the derivative employed and the native sequence being more preferred and at least 90% being very especially preferred. The term functional derivatives covers in particular those amino acid sequences with conservative substitution in comparison with the physiological sequences. The term conservative substitutions refers to those substitutions where amino acids from the same class are exchanged for one another. There are, in particular, amino acids with aliphatic side chains, positively or negatively charged side chains, aromatic groups in the side chains or amino acids whose side chains are capable of forming hydrogen bridges, for example side chains with a hydroxy function. This means that for example an amino acid with a polar side chain is replaced by another amino acid with a likewise polar side chain, or, for example, that an amino acid which is characterized by a hydrophobic side chain is replaced by another amino acid with a likewise hydrophobic side chain (for example serine (threonine) by threonine (serine), or leucine (isoleucine) by isoleucine (leucine)). Insertions and substitutions are possible in particular at those sequence positions which do not bring about a change in the spatial structure or which relate to the binding region. Suitable methods for generating proteins with amino acid sequences which contain substitutions in comparison with the native sequence(s) are disclosed for example in the publications U.S. Pat. No. 4,737,462, U.S. Pat. No. 4,588,585, U.S. Pat. No. 4,959,314, U.S. Pat. No. 5,116,943, U.S. Pat. No. 4,879,111 and U.S. Pat. No. 5,017,691. The generation of derivatives is described in particular also by Sambrook et al, (1989, Molecular Cloning: A Laboratory Manual, Cold Spring Harbor Laboratory Press), it being possible to leave out, complement or substitute codons. Other derivatives may be in particular those proteins which are stabilized in order to avoid physiological degradation, for example by stabilizing the protein backbone by a substitution of by stabilizing the protein backbone by substitution of the amide-type bond, for example also by employing [beta]-amino acids.

Therefore, by using a functional derivative of TLR may lead to an improved capacity of the TLR-domain to bind to MD-2 and/or LPS in the later formed complex. Additionally, it is possible to shorten the extracellular domain of the TLR moiety proceeding from the N- and/or C-terminus to obtain the minimum and/or optimal part necessary for binding LPS in conjunction with MD-2.

On the other hand, it is possible to alter the MD-2-moiety of the fusion protein, for example by introducing different mutations into the nucleic acid sequence coding for the MD-2-moiety which are intended to regulate or improve the formation of the TLR-MD-2 complex or the subsequent formation of the TLR-MD-2-LPS complex. For example, since MD-2 is rich in cysteine residues, formation of polymeric structures could be a problem. It is known, that by mutation of 5 cysteine residues, the polymerization of MD-2 can be inhibited whereby the biological activity is maintained. Also, other types of mutations, such as deletion, insertion, frame shift, nonsense, missense mutations are possible, provided that the capability of the MD-2-domain to form a complex with the TLR-domain and LPS is maintained.

As mentioned above, dimerization or polymerization of TLR-MD-2 fusion proteins can be mediated by an IgG-Fc-domain. Moreover, dimerization or polymerization of the TLR-MD-2 units can be mediated by the TLR extracellular domains of the TLR-moieties of several fusion proteins which may be covalently linked by one or more disulfide bonds. Thus, the effective recombinant TLR-MD-2 fusion protein according to the present invention may also be present in form of a dimer or polymer. It may be present in form of a homodimer or polymer, if identical TLR-MD-2 fusion units are linked, or in form of a heterodimer or polymer, if the complex is formed by different TLR-MD-2 fusion proteins.

The present invention furthermore relates to an nucleic acid molecule encoding a fusion protein as described above. The nucleic acid molecule may be a DNA molecule, e.g. a double-stranded or single-stranded DNA molecule, or an RNA molecule. The nucleic acid molecule may encode the fusion protein or a precursor thereof, e.g. a pro-or pre-proform of the fusion protein which may comprise a signal sequence or other heterologous amino acid portions for secretion or purification which are preferably located at the N-and/or C-terminus of the fusion protein. The heterologous amino acid portions may be linked to the first and/or second domain via a protease cleavage site, e. g.a FactorXa, thrombin orlgA protease cleavage site.

The nucleic acid molecule may be operatively linked to an expression control sequence, e. g. an expression control sequence which allows expression of the nucleic acid molecule in a desired host cell. The nucleic acid molecule may be located on a vector, e. g. a plasmid, a bacteriophage, a viral vector, a chromosal integration vector, etc. Examples of suitable expression control sequences and vectors are described for example by Sambrook et al. (1989) Molecular Cloning, A Laboratory Manual, Cold Spring Harbor Press, and Ausubel et al. (1989), Current Protocols in Molecular Biology, JohnWiley & Sons.

In order to express the biological active recombinant TLR-MD-2 fusion protein of the present invention, the nucleotide sequences encoding the TLR- and MD-2-domains are inserted in frame into an appropriate expression vector, that is, a vector which contains the necessary elements for the transcription and translation of the inserted coding sequences in host cells.

A variety of expression vector/host systems may be utilized to contain and express the TLR-MD-2 fusion protein sequences. These include microorganisms such as bacteria transformed with recombinant bacteriophage, plasmid or cosmid DNA expression vectors, yeast transformed with yeast expression vectors, insect cell systems infected with virus expression vectors or animal cell systems. In principle, all expression systems that involve mammalian, bacterial or fungal cells may be used. For example, the TLR-MD-2 fusion proteins are prepared from permanently transfected *Drosophila melanogaster* S2-cells. After cultivating the host cells, the produced TLR-MD-2 fusion protein or fusion protein polymer complex is recovered from the cell culture.

A further aspect of the present invention relates to a pharmaceutical composition comprising as an active agent at least one fusion protein according to the present invention or a homo- or hereropolymer thereof or a nucleic acid molecule coding for a recombinant fusion protein.

Within the scope of the present invention, the TLR-MD-2 fusion proteins according to the invention, or the other subjects of the present invention, are preferably used in such a way for the production of a pharmaceutical preparation or for the treatment of the abovementioned diseases or disorders that they are suitable for parenteral, i.e. for example subcutaneous, intramuscular, intraarterial or intravenous, or oral or intranasal or anal, intraperitoneal, 1, vaginal or buccal, intracerebral, intraocular administration (injection or infusion), if appropriate also for topical application.

Each of the TLR-MD-2 fusion proteins according to the invention or host cells which form TLR-MD-2 fusion proteins according to the invention, or the DNA sequences which encode recombinant TLR-MD-2 fusion proteins, or suitable expression vectors, can act as medicament per se or be used for the production of a pharmaceutical preparation. However, they may also be employed as medicament in combination with other active ingredient components or pharmaceutical adjuvants, carriers or additives. Thus, the TLR-MD-2 fusion proteins according to the invention or the further subjects of the invention can be combined as constituents in combination with pharmaceutically acceptable carriers, adjuvants and/or additives. Also disclosed in accordance with the present invention are therefore (pharmaceutical) compositions comprising subjects according to the invention, in particular TLR-MD-2 fusion proteins according to the invention. Suitable preparation procedures are disclosed in "Remington's Pharmaceutical Sciences" (Mack Pub. Co., Easton, Pa., 1980), whose contents are herewith incorporated by reference. Suitable carriers for parenteral administration are, for example, sterile water, sterile salines, polyalkylene glycols, hydrogenated naphthalene and, in particular, biocompatible lactide polymers, lactide/glycolide copolymer or polyoxyethylene/polyoxy-propylene copolymers. Compositions according to the invention may comprise fillers or substances such as lactose, mannitol, substances for covalently linking polymers, such as, for example, polyethylene glycol, to inhibitors according to the invention, complexing with metal ions or inclusion of materials in or on specific preparations of polymer compound, such as, for example, polylactate, polyglycolic acid, hydrogel, or on liposomes, microemulsion, micelles, unilamellar or multilamellar vesicles, erythrocyte fragments or spheroplasts. The choice of the individual embodiments of the compositions depends on the physical behavior, for example with regard to solubility, stability, bioavailability or degradability. Controlled or constant release of the active ingredient component according to the invention in the composition includes formulations based on lipophilic depots (for example fatty acids, waxes or oils). Coatings of substances according to the invention or compositions comprising such substances, viz. coatings with polymers, are likewise disclosed within the scope of the present invention (for example poloxamers or poloxamines). Furthermore, substances or compositions according to the invention may be provided with protective coatings, for example protease inhibitors or permeabilizing agents.

With the inventive TLR-MD-2 fusion proteins there are provided competitive ligand-binding soluble receptors which are able to moldulate signaling of TLRs by competing for LPS and therefore can be used as active ingredients in pharmaceutical compositions for the prevention or treatment of allergic reactions or inflammatory conditions, such as Gram-positive and Gram-negative sepsis, in mammals.

### Examples

### Materials and Methods

### Cell culture

RAW 264.7 were cultured in RPMI 1640 (PAN Biotech, Aidenbach, Germany) containing 10% fetal calf serum at 37°C in humidified air with 5% CO₂. Human embryonic kidney (HEK) 293 cell line were grown in Dulbecco's modified eagle medium (DMEM; PAA Laboratories, Linz, Austria) supplemented with 10% fetal calf serum at 37°C in humidified air with 10% CO₂. S2 cells were cultured in Insect Xpress medium (BioWhittaker, Cambrex, Verviers, Belgium) at 21°C.

### Constructs

The extracellular domains of mouse TLR2 and TLR4 (GeneBank AF216289; nt:389-2067; mTLR4 GenBank: AF110133; nt: 85 -1902), excluding the signal sequences, were amplified by polymerase chain reaction using *Pfu-*polymerase (Stratagene, Heidelberg, Germany) using as templates cDNA prepared from LPS-stimulated RAW 264.7 cells (for TLR2) or a plasmid containing Flag-mTLR4 (a kind gift of Bruce Beutler, Scripps, La Jolla, CA), respectively. The 5' primer introduced a Flag-tag into the TLR2 construct. These fragments were subcloned into the pSignal IG plus vector (R&D Systems, Wiesbaden, Germany) in frame with the C-terminal human IgG1 Fc to yield FlagmrsTLR2-IgGFc (T2Fc)- and FlagmrsTLR4-IgGFc (T4Fc)-encoding cDNAs. These cDNAs were excised and cloned into the pMT/BiP/V5-His B vector (Invitrogen, Karlsruhe, Germany), containing a metallothionin promoter and fused in frame to the BiP signal-sequence. For expression in mammalian HEK 293 cells mrsTLR4-IgGFc was cloned into expression vector p3xFlag-CMV-8 (Sigma, Deisenhofen, Germany).

The coding sequence of murine MD-2 cDNA, excluding the signal sequence (GenBank: AB018550; nt:101-535) was amplified by PCR and the fragment was cloned into pFlag-CMV-1 expression vector (Sigma) in frame with the Flag-tag.

### Expression and purification of fusion proteins

Exponentially growing S2 cells were cotransfected with pMT/BiP/V5-His B expression vector containing T2Fc or T4Fc, respectively, and pCoHygro (Invitrogen) using Fugene 6 transfection reagent (Roche Diagnostics, Mannheim, Germany) following the manufacturers instructions. After 3 days, the cells were centrifuged and resuspended in Insect Xpress medium containing 300 µg/ml hygromycin B (Roche Diagnostics). The medium was replaced every 5 days. Stably transformed polyclonal cell populations were isolated after 4 weeks of selection with hygromycin B. Hygromycin B was maintained routinely in the media at all times after selection. Five days after induction with CuSO₄ (500 µM), protein production was analyzed by SDS-PAGE and Western blot analysis.

Culture supernatants containing T2Fc or T4Fc (200 ml), respectively, were dialyzed against PBS and purified by Protein A Agarose affinity chromatography (Affi-Gel Protein A; Bio-Rad Laboratories, Munich, Germany). Bound fusion protein was eluted with 2 ml aliquots of 0.1 M Glycin/HCl buffer at pH 4 and neutralized immediately. Proteins were then dialyzed extensively against PBS and stored at 4°C.

HEK 293 cells (3x10⁶) were transfected by the calcium phosphate technique, using 10 µg of the indicated plasmids per 100-mm² dish. Six hours after transfection, cells were washed once with PBS and 5 ml fresh serum-free medium was added. The cells were harvested after an additional 48 h of culture.

Supernatants (20 ml) from transfected HEK 293 cells containing T4Fc and MD-2, were concentrated by centrifugation using a size exclusion membrane with a cut-off of 10 kDa (Centricon YM-10 concentrators Millipore, Schwalbach, Germany), yielding 2 ml. Control supernatants (Mock) were prepared in the same way, except that the plasmids were omitted.

### Immunoprecipitation

Supernatants of transfected HEK 293 cells (3 ml) or S2 cells (1 ml), were subjected to IP with 30 µl Protein G Sepharose (Amersham Biosciences, Freiburg, Germany) for 2-10 h. Immunocomplexes were washed, boiled in Laemmli reducing buffer and resolved by SDS-PAGE.

### SDS-PAGE, Western blot, protein analysis and factor Xa digestion

Protein samples were analyzed by reducing (boiled in SDS sample buffer with Dithiothreitol, (DTT)) and nonreducing (boiled in SDS sample buffer without DTT) 4-12% SDS-PAGE (Nupage Bis-Tris-Gel; Invitrogen) and detected either directly by Syprored protein stain (Molecular probes, Leiden, The Netherlands) or by Western blot analysis after electroblotting onto nitrocellulose membranes (Optitran BA-S 83 Reinforced NC; Schleicher and Schuell, Dassel, Germany). After blotting, membranes were blocked with 5% fat-free milk powder in PBS containing 0,02% Triton X-100 (Sigma). Proteins were detected with anti-Flag mAb (M2) biotin conjugate (Sigma), followed by Streptavidin horseradish peroxidase (Amersham Biosciences) and developed by using the enhanced chemiluminescence kit (ECL, Amersham Biosciences). Alternatively by using anti-Flag mAb (M2) (Sigma), followed by an alkaline phosphatase conjugated anti-mouse IgG (whole molecule) secondary antibody. Specific bands were revealed using Nitro blue tetrazolium chloride/ 5-Bromo-4-chloro-3-indolyl phosphate, toluidine salt (NBT/BCIP) -system (Roche Diagnostics).

Protein concentration of consecutive fractions from Protein A Agarose affinity chromatography was measured using Nano Orange protein quantitation kit (Molecular probes) with BSA as standard.

For factor Xa digestion, purified T2Fc or T4Fc were dialyzed against digestion buffer containing 100 mM NaCl, 50 mM Tris, and 1 mM CaCl₂, pH 8. Dialyzed fusion proteins were incubated for 24 h at room temperature with protease factor Xa (Roche Diagnostics), 1/20 of the substrate by weight. Fc fragments and remaining undigested proteins were removed by passage through Protein A Agarose.

### LPS binding assay

LPS was biotin-labeled using biotin hydrazide (Pierce, Perbio, Bonn, Germany) according to the manufacturers instructions. Successful biotinylation of LPS was determined by SDS-PAGE and Western blot using horseradish peroxidase-conjugated anti-biotin antibody (Sigma). The binding of proteins to LPS was examined by incubating supernatants (3 ml) from transfected HEK 293 cells with biotinylated LPS (1 µg for 1 h at 37 °C followed by IP with 30 µl Streptavidin sepharose (Amersham Biosciences). Bound protein was detected by Western blot.

### Cell stimulation assay, ELISA

RAW 264.7 were seeded in 96-well plates (Costar, Cambridge, MA) at a density of 10⁶ cells/ml in the presence or absence of the indicated proteins and stimulated with different concentrations of Pam3Cys or purified LPS from *Salmonella abortus equi* S-form (both reagents were from Alexis Biochemicals, Grünberg, Germany), overnight in 0.1 ml medium. The supernatants were tested for IL-6 by ELISA, according to the manufactures instructions (Becton Dickinson, Heidelberg, Germany).

### Example 1

### Expression and purification of T2Fc and T4Fc

In order to investigate the role of soluble TLRs in TLR signaling and their influence on signaling via membrane-bound TLRs, soluble chimeric TLR2 and TLR4 fusion proteins were produced. Since dimerization seems to be the minimal requirement for TLR2 and TLR4 to function as signal transducers, recombinant fusion proteins between the entire extracellular domain of TLR2 or TLR4 and human IgG1-Fc (T2Fc and T4Fc), containing an N-terminal Flag-tag, were produced in S2-cells.

High level protein production and secretion was induced in stably transfected cells with 500 µM CuSO₄ in serum-free medium, resulting in accumulation of the recombinant products in the conditioned medium. Production was monitored by Immunoprecipitation (IP) of 1 ml conditioned medium with Protein G Sepharose following Western blot analysis using an anti-Flag mAb. Analysis under reducing conditions revealed the Flag-tagged monomers with an approximate molecular size of 105 kDa (T2Fc) and 125 kDa (T4Fc) (Fig. 2).

Under non-reducing conditions Western blotting presented the expected dimerized receptors with an approximate molecular size of 210 kDa (T2Fc) and 250 kDa (T4Fc) (data not shown). For further biochemical characterization fusion proteins were cleaved by protease factor Xa to remove the Fc-tail. Western blotting showed the expected polypeptide bands of approximately 80 kDa (T2Fc) and 100 kDa (T4Fc) (data not shown).

For efficient purification dialyzed culture media, containing T2Fc or T4Fc, were loaded onto a Protein A Agarose column. Elution was carried out with Glycin/HCl buffer at pH 4 and protein purity was analyzed by SDS-PAGE under reducing conditions using Syprored protein stain. T2Fc and T4Fc fractions displayed a single-band with an approximate molecular weight of 105 kDa (T2Fc) and 125 kDa (T4Fc), respectively. The protein concentration in the peak elution fractions was between 50-200 µg/ml indicating a concentration in the supernatant of up to 10 µg/ml. Thus, highly purified T2Fc and T4Fc using a one step purification scheme were prepared.

### Example 2

### Effects of T2Fc and T4Fc on stimulation of TLR2 and TLR4

To evaluate the functional consequences of T2Fc interference, IL-6 production by RAW 264.7 cells was tested in the presence of different concentrations of the fusion protein. In the presence of T2Fc, but not T4Fc, a dose-dependent positive synergistic effect on Pam3Cys- or LTA -stimulated cells was found. This synergy was not due to the fused IgGFc or the dimerization, since removal of IgGFc by factor Xa digestion did not abolish this effect. T2Fc or T4Fc alone had no measurable effect on RAW 264.7 IL-6 secretion, indicating minimal or no LPS contamination. In addition, T2Fc had no influence on LPS-stimulated RAW 264.7.

To determine the function of T4Fc, IL-6 concentrations were measured after stimulation of RAW 264.7 with purified LPS. Although a very broad dose range of T4Fc was tested (0,001-6 µg/ml) T4Fc had no influence on IL-6 levels (Fig. 3). The same result was obtained when the Fc fragment was removed by factor Xa digestion.

### Example 3

### Role of MD-2

Since MD-2-binding to TLR4 is required for the assembly of a functional LPS receptor, the binding of T4Fc to MD-2 was assessed. T4Fc and MD-2 were therefore co-expressed in HEK 293 cells and supernatants immunoprecipitated with Protein G Sepharose. After IP, T4Fc and MD-2 were identified based upon their molecular weights by Western blot using an anti-Flag mAb. MD-2 was coprecipitated with T4Fc, demonstrating physical interaction of T4Fc with MD-2.

To further determine whether this complex recognized LPS, direct interaction between LPS and T4Fc/MD-2 using a "LPS Pull-down assay" was investigated.

Supernatants of HEK 293 cells, transfected with T4Fc and MD-2 either alone or in combination were incubated with biotinylated LPS (Biotin-LPS) and immunoprecipitated with Streptavidin Sepharose. The resultant immunoprecipitates were subjected to SDS-PAGE and immunoblotted with an anti-Flag mAb. When T4Fc and MD-2 were co-expressed two detectable bands corresponding to T4Fc and MD-2 were found, demonstrating that LPS was associated with T4Fc and MD-2. T4Fc alone did not interact with Biotin-LPS, whereas MD-2 alone was immunoprecipitated with Biotin-LPS, indicating a direct interaction between MD-2 and LPS but not between T4Fc and LPS.

Thus, the above co-IP experiments with MD-2 and LPS demonstrate intact biological activity of T4Fc and a suitable conformation of the T4Fc/MD-2 complex.

### Example 5

### Inhibitory activity of T4Fc/MD-2 complex

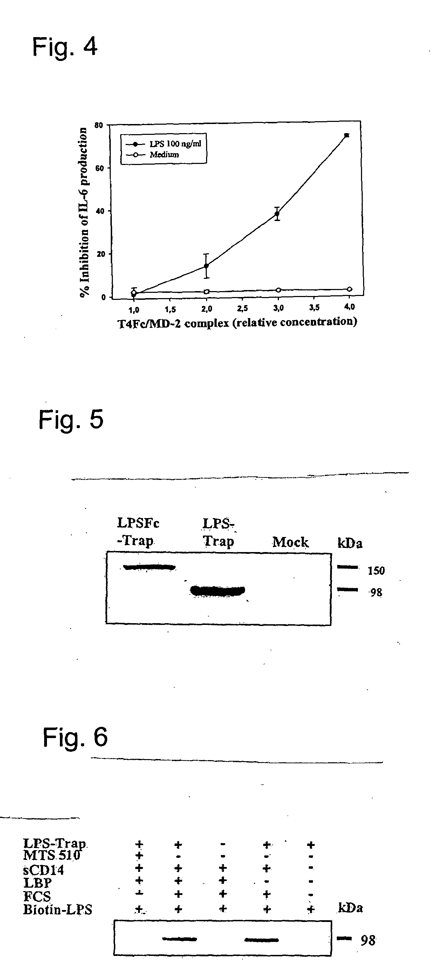

Since the T4Fc/MD-2 complex was able to bind LPS, it was next investigated whether it had any inhibitory capacity *in vitro.* HEK 293 cells were cotransfected with T4Fc and MD-2 and the supernatant was concentrated by using size exclusion filtration. Mock supernatants were prepared and analyzed in parallel. Fractions of the concentrates were analyzed by SDS-PAGE and Western blotting with an anti-Flag mAb. The results showed the expected bands for T4Fc and MD-2 which were not present in the concentrated mock supernatants. The concentrated supernatants were added to RAW 264.7 cells 30 minutes prior to LPS-stimulation. Fig. 4 shows a clear dose-dependent inhibition of IL-6 production when T4Fc/MD-2 complex was added in contrast to mock concentrated supernatant. T4Fc/MD-2 complex alone showed no activity. Therefore, it can be concluded, that the T4Fc/MD-2 complex has the capacity to bind and neutralize LPS *in vitro.*

### Example 6

### Preparation of a recombinant TLR4-MD-2 fusion protein capable of binding and neutralizing LPS (LPS-Trap)

Since a T4Fc/MD-2 complex has the capacity to bind and neutralize LPS *in vitro,* a preformed TLR4-MD-2 fusion protein was created. For this purpose, the extracellular part of TLR4 was fused with MD-2 via a flexible linker.

The c-terminus of the construct was provided with a Fc-tail of a IgG *(LPS-Trap-Fc)* which can mediate the dimerization of TLR4-MD-2 fusion proteins and enables the detection and/or isolation of the fusion proteins. Alternatively, a HIS₆-tail for facilitating the detection and/or isolation of the fusion proteins was attached to the C-terminus of the construct *(LPS-Trap).*

The fusion proteins *LPS-Trap* and *LPS-Trap-Fc* were derived from S2-cells stably transfected with cDNA-constructs encoding the TLR4-MD2-fusion proteins. The supernatants of transfected S2-cells were immunoprecipitated with Flag-agarose and analysed by Western blot with an Anti-Flag mAb (Fig. 5). The Western Blot analysis shown in Figure 5 verifies the expression of *LPS-Trap-Fc* and *LPS-Trap* in S2-cells. Moreover, the biological activity, i. e., the LPS-binding capacity, of the constructs was tested *in vitro.* For that purpose, the TLR4-MD-2-fusion protein LPS-*Trap* was incubated with different serum proteins, added with biotinylated LPS and thereafter immunoprecipitated with streptavidin-sepharose. To demonstrate the specific binding of LPS to the TLR4-MD-2-fusion protein, it was preincubated with neutralizing mAb MTS 510 (mAB TLR4/-MD-2). The Anti-flag Western-blot analysis revealed the binding of LPS to *LPS-Trap* which is neutralized by the TLR4/MD-2 antibody (Fig. 6).

## Claims

1. A recombinant TLR-MD-2 fusion protein capable of binding to lipopolysaccharide (LPS) containing a TLR-moiety comprising the extracellular domain of a Toll-like receptor (TLR) or a functional derivative thereof, fused with a MD-2-moiety comprising MD-2 or a functional derivative thereof.

2. A recombinant TLR-MD-2 fusion protein according to claim 1, **characterized in that** the TLR-moiety and MD-2-moiety are fused via a flexible linker.

3. A recombinant TLR-MD-2 fusion protein according to claim 1, **characterized in that** the TLR-moiety and MD-2-moiety are fused via an inflexible linker.

4. A recombinant TLR-MD-2 fusion protein according to any one of the preceding claims, **characterized in that** the Toll-like receptor moiety is the extracellular domain of TLR4 or a functional derivative thereof.

5. A recombinant TLR-MD-2 fusion protein according to any one of claims 1 to 3, **characterized in that** the Toll-like receptor moiety is the extracellular domain of TLR2 or a functional derivative thereof.

6. A recombinant TLR-MD-2 fusion protein according to any one of the preceding claims, **characterized in that** at least one of the TLR-moiety and the MD-2-moiety is fused with a Fc-domain of human IgG.

7. A recombinant TLR-MD-2 fusion protein according to claim 6, **characterized in that** at least one of the TLR-moiety and the MD-2-moiety is fused with the Fc-domain of human IgG1.

8. A recombinant TLR-MD-2 fusion protein according to any one of the preceding claims, **characterized in that** at least one of the TLR-moiety and the MD-2-moiety is fused with a HIS-domain.

9. A recombinant TLR-MD-2 fusion protein according to any one of the preceding claims, **characterized in that** the TLR-moiety comprises a mutated form of the extracellular domain of the Toll-like receptor.

10. A recombinant TLR-MD-2 fusion protein according to any one of the preceding claims, **characterized in that** the MD-2-moiety comprises a mutated form of the MD-2 protein.

11. A recombinant TLR-MD-2 fusion protein polymer consisting of two or more dimerized or polymerized TLR-MD-2 fusion proteins according to claim 1 to 10.

12. A recombinant TLR-MD-2 fusion protein polymer according to claim 11, **characterized in that** the complex is formed by identical TLR-MD-2 fusion protein units.

13. A recombinant TLR-MD-2 fusion protein complex according to claim 11, **characterized in that** the complex is formed by different TLR-MD-2 fusion protein units.

14. A nucleic acid molecule, **characterized in that** it encodes a recombinant fusion protein or fusion protein complex according to claim 1 to 13.

15. An expression vector, **characterized in that** the expression vector comprises a nucleic acid sequence according to claim 14.

16. A host cell, **characterized in that** the host cell is transfected with an expression vector according to claim 15.

17. A method for preparing the TLR-MD-2 fusion protein or fusion protein complex of claim 1 to 13, comprising
inserting the nucleic acid sequences coding for the recombinant TLR-MD-2 fusion protein or fusion protein complex according to claim 1 to 13 into an expression vector,
transfecting said expression vector into host cells,
recovering the produced TLR-MD-2 fusion protein (complex) from the cell culture.

18. A method according to claim 17, **characterized in that** the TLR-MD-2 fusion protein is recovered from stably transfected *Drosophila melanogaster* S2 cells.

19. A pharmaceutical composition containing a TLR-MD-2 fusion protein or fusion protein complex of any one of claims 1 to 13 as active ingredient.

20. A pharmaceutical composition according to claim 19 for the preventive and/or therapeutic treatment of allergic and inflammatory conditions.

21. A pharmaceutical composition according to claim 20 for the preventive and/or therapeutic treatment of sepsis.
